# EUROPEAN PATENT APPLICATION

(11) **EP 2 098 503 A1**
(43) Date of publication of application: **09.09.2009**
(21) Application number: 07817119.6
(22) Date of filing: 19.10.2007
(51) Int. Cl.: C07C 311/39, C07C 303/40, A61K 31/18, A61K 9/20, A61K 9/14, A61K 9/48, A61K 9/08, A61P 13/08

(54) **5-[(2R)-[2-[2-[2-(2,2,2-TRIFLUOROETHOXY)PHENOXY]ETHYL]AMINO]PROPYL]-2-METHOXYBENZENESULFONAMIDE**

(30) Priority: 30.11.2006 CN 200610163317
(71) Applicant: Jiangsu Hansen Pharmaceutical Co., Ltd., Jiangsu 222047 (CN)
(72) Inventor: ZHONG, Huijuan, Jiangsu 222047 (CN); CEN, Junda, Jiangsu 222047 (CN)
(74) Representative: Hart-Davis, Jason
(86) International application number: PCT/CN2007/070928
(87) International publication number: WO 2008/064595

(57) **Abstract**

5-[(2R)-[2-[2-[2-(2,2,2-trifluoroethoxy)phenoxy]ethyl]amino]propyl]-2-methox ybenzenesulfonamide, a pharmaceutical composition containing the compound, and the synthesis method thereof. The compound has strong antagonism toward α1-adrenceptor and has high selectivity toward smooth muscle of urethra.

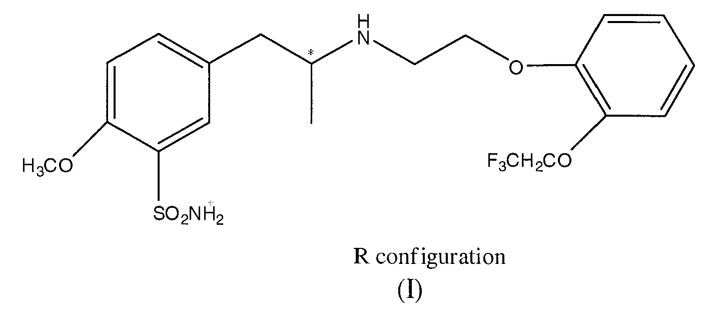

## Description

### FIELD OF THE INVENTION

The present invention relates to a 5-[(2R)-[2-[2-[2-(2,2,2-trifluoroethoxy) phenoxy]ethyl]amino]propyl]-2-methoxybenzenesulfonamide, its pharmaceutical acceptable salts and a method for preparing the same; it also provides a pharmaceutical composition containing the compound and use thereof in the preparation of an anti-prostatauxe medicament.

### BACKGROUND OF THE INVENTION

Prostatic hyperplasia is a common disease in the elderly, its main clinical symptom is dysuria. At present, adrenal α1 receptor antagonists are usually used to alleviate the symptom, such as prazosin (U.S. Patent US3,511,836), terazosin (U.S. Patent US4,026,894), doxazosin (U.S. Patent US4,188,390), alfuzosin (U.S. Patent US4,315,007), Tamsulosin (U.S. Patent US4,703,063) and so on, these drugs selectively inhibit adrenal α1 receptor and the contraction of smooth muscle of urethral, then urine is well excreted, but these drugs are associated with side effects, such as orthostatic hypotension, so they should be used carefully to patients.

Therefore, in order to treat dysuria, it is desirable to find a compound which would selectively inhibit the contraction of smooth muscle of urethral and weakly affect the contraction on blood vessel. The compound of the present invention has a higher selectivity in inhibiting the contraction of smooth muscle of urethral and can be used in the treatment for dysuria, moreover the incidence of orthostatic hypotension is lower.

### DESCRIPTION OF THE INVENTION

The object of the present invention is to provide a new compound as an adrenal α1 receptor antagonist.

Another object of the present invention is to provide a preparation process of a new compound having formula (I).

The present invention also provides a pharmaceutical composition containing a therapeutically effective dose of a compound having formula (I).

Further, the present invention also aims to provide the use of a compound having formula (I) and a composition containing it in the preparation of an anti-prostatauxe medicament.

In order to complete this invention, the present invention relates to the following technical solutions:
According to this invention, it specially relates to a pharmaceutical acceptable salt of a compound having formula (I).

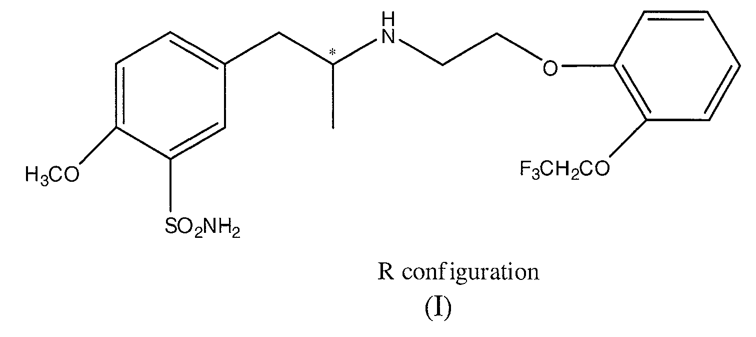

The present invention also relates to a preparation process of the compound having formula (I), which comprises the steps of:
reacting the compound of formula (II) with the compound of formula (III) to give the compound as described in this invention.

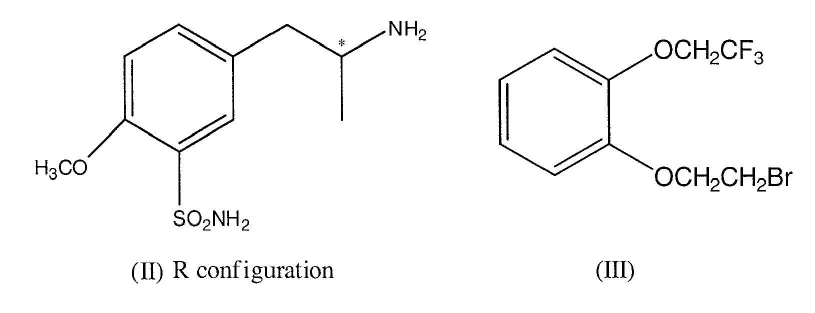

The present invention also involves a pharmaceutical composition characterized in containing a therapeutically effective dose of the compound as active ingredient and the pharmaceutical acceptable carriers.

The compound of this invention can be used in the preparation of an anti-prostatauxe medicament.

It can be seen that the present invention relates to a compound having formula (I) or its pharmaceutical acceptable salt. It also relates to a pharmaceutical composition of the compound, which has excellent activity of anti-prostatic hypertrophy.

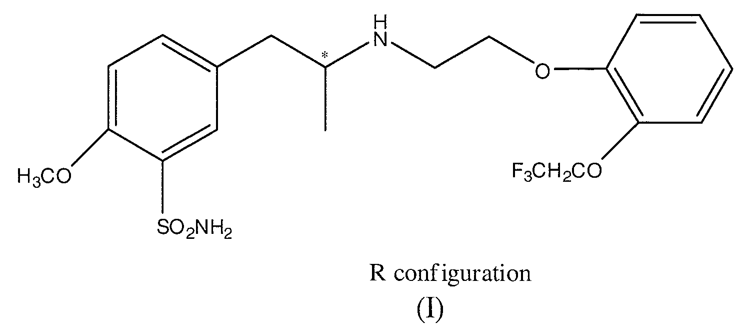

It has been reported that some adrenal α1 receptor antagonists have a good inhibitory effect on the contraction of smooth muscle of urethral and are used in the treatment for prostatic hyperplasia. The present inventors found that the compound having formula (I) has stronger pharmacological effects and better selectivity compared with the reported drugs, so they completed the present invention.

A compound having formula (I) can be prepared by reacting a compound of formula (II) with a compound of formula (III).

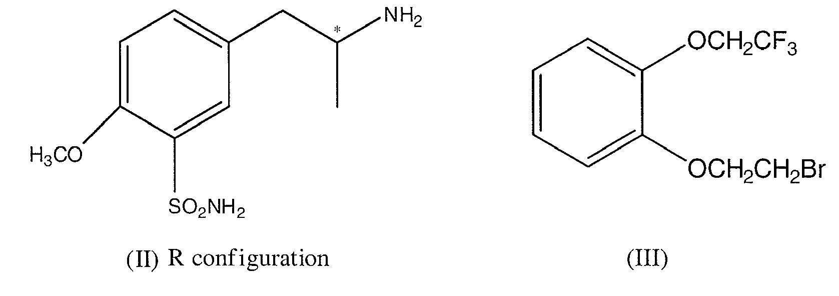

Typically, a pharmaceutical composition of the invention can be prepared by a method known in the art. In order to have this object, if necessary, active ingredients can be combined with one or more solid or liquid pharmaceutical excipients and/or adjuvants, then further made into suitable used form or dosage form as a human medicine.

A pharmaceutical composition of the invention can be administered in the form of unit-dose, and the routes of administration may be intestinal or parenteral, such as oral, muscle, hypodermic, nasal cavity, oral mucosa, skin, peritoneum, or rectum and so on.

A composition of the present invention can be administered by injection. Said Injection includes intravenous injection, intramuscular injection, subcutaneous injection, intra-dermal injection, acupuncture points injection and so on.

Dosage form of administration can be liquid and solid dosage forms. Liquid forms can be a true solution, colloid, particulate, emulsion, suspension. Other dosage forms are tablets, capsules, drop pills, aerosols, pills, powders, solutions, suspensions, emulsions, particulates, suppositories, freeze-dried powders and so on.

A pharmaceutical composition of the present invention can be made into general preparations, or delayed-release preparations, controlled-release preparations, targeting preparations and various particulate preparations.

In order to prepare unit-dose into tablets, the various carriers known in the art can be used widely. Some examples of carriers include, for example diluents and absorbents, such as starch, dextrin, calcium sulfate, lactose, mannitol, sucrose, sodium chloride, glucose, urea, calcium carbonate, kaolin, microcrystalline cellulose, aluminum silicate, etc.; wetting agents and binders, such as water, glycerol, polyethylene glycol, ethanol, propanol, starch, dextrin, syrup, honey, glucose solution, mucilago acaciae, gelatine solution, sodium carboxymethyl cellulose, gum lac, methyl cellulose, potassium phosphate, polyvinylpyrrolidone, etc.; disintegrants, such as dry starch, alginate, agar powder, brown algae starch, sodium bicarbonate and citric acid, calcium carbonate, polyoxyethylene sorbitol fatty acid ester, sodium dodecyl sulfate, methyl cellulose, ethyl cellulose, etc.; disintegration inhibitors, such as sucrose, tristearic acid glyceride, cocoa butter, hydrogenated oil etc.; absorption enhancers, such as the quaternary ammonium salt, sodium dodecyl sulfate etc.; lubricants, such as talcum powder, silica, corn starch, stearate, boric acid, liquid paraffin, polyethylene glycol, etc.. Said tablets can also be further prepared into coated tablets, such as sugar coated tablets, film coated tablets, enteric coated tablets, or double-layer tablets and multi-layer tablets.

In order to prepare administration unit into pills, the various carriers known in the art can be used widely. Some examples of carriers include, for example diluents and absorbents, such as glucose, lactose, starch, cocoa butter, hydrogenated vegetable oil, polyvinylpyrrolidone, kaolin, talc, etc.; binders, such as arabic gum, bassora gum, gelatin, ethanol, honey, liquid sugar, rice paste or panada, etc.; disintegrants, such as agar powder, dry starch, alginate, sodium dodecyl sulfate, methyl cellulose, ethyl cellulose etc..

In order to prepare administration unit into suppositories, the various carriers known in the art can be used widely. Some examples of carriers include, such as polyethylene glycol, lecithin, cocoa butter, higher alcohols, esters of higher alcohols, gelatin, semi-synthetic glyceride, etc..

In order to prepare administration unit into capsules, the active ingredient is mixed with the above various carriers, and then the mixture are placed in hard gelatin capsules or soft capsules. The active ingredient can also be prepared into encapsulated formulation, be suspended in aqueous medium to form suspension, be put into a hard capsule or be prepared into injection for use.

For example, the composition of the present invention is prepared into injection, such as solutions, suspensions, emulsions, freeze-dried powders, which may be aqueous or non-aqueous, and contain one or more pharmaceutical acceptable carriers, diluents, binders, lubricants, preservatives, surfactants or dispersants. For example the diluents may be selected from the group consisting of water, ethanol, polyethylene glycol, 1,3-propanediol, ethoxylated isooctadecanol, peroxidized isooctadecanol, polyoxyethylene sorbitol fatty acid esters etc.. In addition, in order to prepare isotonic injection, a suitable amount of sodium chloride, glucose or glycerol can be added into the injection, moreover, regular cosolvents, buffers, pH adjusting agents can also be added. These excipients are generally used in this art.

Furthermore, if necessary, coloring agents, preservatives, perfumes, correctives, sweeteners or other materials may be added into the pharmaceutical preparations.

The pharmaceutical composition of the invention can be used in the treatment for prostatic hyperplasia.

The administered dosage of the invention compound or its pharmaceutical composition depends on many factors, such as the nature and severity of the diseases needed to prevent or treat, sex, age, weight, temperament and individual response of the patients or animals, routes of administration, times of administration, so the therapeutic dose of the present invention has a large-scale changes. Generally speaking, the used dosage of pharmaceutical components of the present invention is known to a person skilled in the art. According to the actual effective dosage contained in the final preparation of compounds or their combination of the present invention, it can be appropriately adjusted to achieve the requirement of its therapeutic effective dose. For the patients with the weight about 75 kg, the dose administrated per day may be in the range of 0.5 µg/kg body weight to 40 µg/kg body weight, preferably in the range of 2 µg/kg body weight to 10 µg/kg body weight. The above dose may be administered in the form of single dose or multidoses, such as two, three or four doses, which depends on the dosage regimen of the doctor on the basis of clinical experience.

The compound having formula (I) prepared by the invention has a strong inhibitory effect and good selectivity on the contraction of smooth muscle of urethra.

### EXAMPLES

The following examples are provided to illustrate the present invention in detail; however, these should not be considered as limiting the scope of this invention.

### Example 1

### Preparation of 5-[(2R)-[2-[2-[2-(2,2,2-trifluoroethoxy)phenoxy]ethyl]amino] propyl]-2-methoxybenzenesulfonamide (a compound of formula I) and its hydrochlorate

A solution of (R)-(-)-5-(2-aminopropyl)-2-methoxybenzenesulfonamide (to be prepared according to the method of the European Patent EP 257787) (24.4g, 0.1 mol ), 2-(2,2,2-trifluoroethoxy-1)phenyl-2-ether bromide (to be prepared according to the method of United States Patent US 5,387,603) (15g, 0.05 mol ) and isopropanol 300ml were refluxed for 16 hour, cooled to 30 °C (centigrade) and filtered. The filtrate was dried under reduced pressure, ethyl acetate 300ml and water 100ml were added, then the mixture was shaken. The ethyl acetate layer was separated, the water layer was extracted with ethyl acetate 100ml, then the combined ethyl acetate layer was washed with 100ml water, dried and evaporated under reduced pressure to give the compound having formula (I) 16g, yield 69% (the hydrochlorate of the compound having formula (I)).

The resulting compound (16g) was dissolved in 150ml absolute ethyl alcohol, then HCl-ethanol was dropwise added to pH2. The mixture was stirred for 0.5 hour, filtered, washed with ethanol and dried to give a white solid 16g, yield of 93% (the hydrochlorate of the compound having formula (I)).

¹HNMR (d₆-DMSO) δ 1.17 (3H , d), 2.73 (1H , d. d) , 3.31 (1H , d. d) , 3.44 (2H , m) , 3.52 (1H , m) , 3.90 (3H , s) , 4.37 (2H , t) , 4.72 (2H , q) , 7.00∼ 7.19 (5H , m) , 7.46 (1H , d. d) , 7.64 (1H , d) , 9.35 (2H , br. s)

### Example 2

**Preparation of tablets of compound I**

| | |
|---|---|
| Compound 1 | 0.1g |
| Starch | 100g |
| Starch slurry (8%) | Proper amount |
| Magnesium stearate | 0.4g |

The resulting compound of Example 1 was mixed uniformly with starch, 8% starch slurry was added to prepare softmaterials, then the softmaterials were granulated with 14 mesh nylon sieve, dried at 70-80°C, magnesium stearate was added, granulated with 10-12 mesh wire sieve, mixed, pressed into tablet with 12mm punch die.

### Example 3

**Preparation of particles of compound 1**

| | |
|---|---|
| Compound 1 | 0.1g |
| Soluble starch | 80g |
| Powdered sugar | 20g |
| Flavors | Proper amount |

The compound 1 was dissolved in the water, and starch (80g), powdered sugar (20g), flavors (proper amount) were added, mixed uniformly, then granulated with 14-16 mesh sieve , dried at a temperature below 60 °C, and packed.

### Example 4

**Preparation of capsules of compound 1**

| | |
|---|---|
| Compound 1 | 0.1g |
| Microcrystalline cellulose | 40g |
| Lactose | 60g |
| Hydroxymethyl starch sodium | 4g |
| Starch slurry | Proper amount |
| Magnesium stearate | 1g |
| Silica gel powder | 1g |

Compound 1, microcrystalline cellulose, lactose and hydroxymethyl starch sodium were sieved respectively and mixed uniformly, then proper starch slurry was added to prepare softmaterials, then the softmaterials were granulated with 20 mesh sieve, the wet particles were dried at a temperature below 50°C, then the dried particles were granulated with 20 mesh sieve, then mixed uniformly with magnesium stearate and silica gel powder, and filled into capsules.

### Example 5

**Preparation of oral solutions of compound 1**

| | |
|---|---|
| Compound 1 | 0.1g |
| Saccharin | 0.5g |
| Flavors | 0.1g |
| Water for injection | 1000ml |

Compound 1, saccharin and flavors were dissolved respectively in water for injection and mixed, then diluted to 1000ml, and subpackaged to obtain oral solutions of compound 1.

### Example 6

**Preparation of injectable powders of compound 1**

| | |
|---|---|
| Compound 1 | 0.1g |
| Dextran | 40g |

Compound 1 was dissolved with proper amount of water for injection, dextran (40g) was also dissolved with proper amount of water for injection, and then the two solutions were mixed, diluted with water for injection to 2000ml, filtered with 0.22µm millipore filter. Under aseptic conditions, the filtrate were subpackaged in 10ml Xilin bottles (vial), packed into disk, and freeze-dried in freeze-dried box, then carried out form the box, and rolled covers.

### Test Example 1

The antagonism and antagonistic selectivity of α1 receptor of different isolated smooth muscle specimens treated with the compound having formula (I).

Methods: a 24-hour fasting rat was hit on the head to induce coma, and quickly dissected the abdominal cavity, its urethra, aorta and vas deferens were respectively taken out and recorded the contraction curve with instruments, then the different concentrations and volumes of norepinephrine were added to get noradrenalin's accumulation curve, rinsed immediately to return to baseline, then different concentrations of test drugs were added. Noradrenaline was added repeatedly after 5 ∼ 8 minute to obtain average contractile response curve of noradrenalin under different concentrations of antagonist, the contraction pD₂ of noradrenaline on the urethra, aorta, vas deferens under different concentrations of samples for test was calculated, the pA₂ value for antagonism of contraction caused by the samples against norepinephrine was obtain by regressing with the concentration of test samples and pD₂.

Results: pA₂ value for the contraction of the different organizations inhibition of smooth muscle specimens induced by the hydrochlorate of compound I and tamsulosin hydrochloride against norepinephrine is shown in Table 1. The intensity ratio of contraction inhibitive effect of urethra and aorta, urethra and vas deferens smooth muscle, caused by the sample against noradrenaline is disclosed in table 2, which is calculated by pA₂ value.

Table 1 pA₂ value for the contraction of the different organizations of smooth muscle specimens induced by the hydrochlorate of compound I and tamsulosin hydrochloride inhibiting norepinephrine

| Sample | pA₂ value for the contraction caused by inhibiting norepinephrine | | |
|---|---|---|---|
| | urethra | aorta | vas deferens |
| Tamsulosin hydrochloride | 9.22 | 8.63 | 10.12 |
| the hydrochlorate of compound I | 11.00 | 8.21 | 10.08 |

Table 2 The intensity ratio of the contraction of the different organizations of smooth muscle specimens induced by the hydrochlorate of compound I and tamsulosin hydrochloride inhibiting norepinephrine

| Sample | the intensity ratio of the contraction caused by inhibiting norepinephrine | |
|---|---|---|
| | urethra/ aorta | urethra/ vas deferens |
| Tamsulosin hydrochloride | 3.83 | 0.123 |
| the hydrochlorate of compound I | 607.31 | 8.120 |

## Claims

1. A compound having formula (I) or a pharmaceutical acceptable salt thereof.

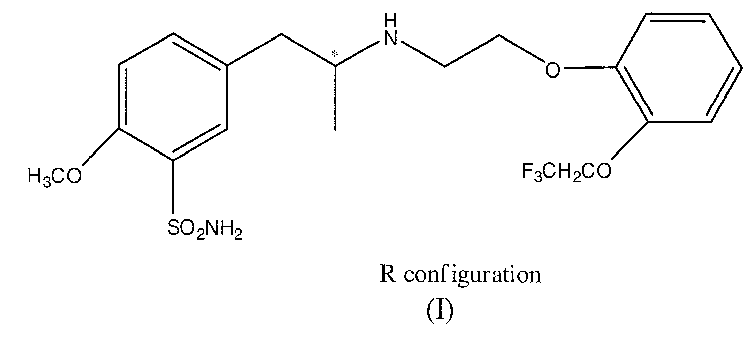

2. A preparation process of the compound of claim 1, comprising the steps of reacting the compound of formula (II) with the compound of formula (III).

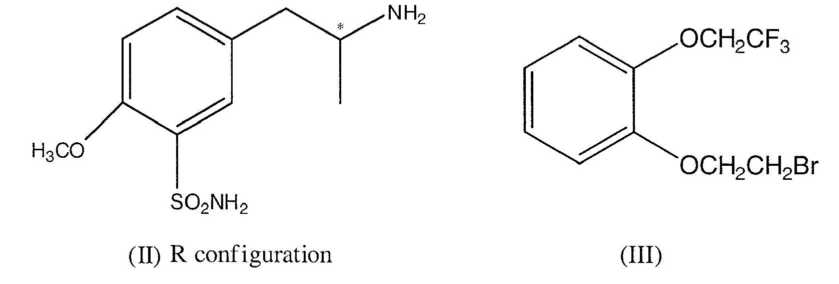

3. A pharmaceutical composition, **characterized in** comprising the compound or the pharmaceutical acceptable salt thereof of claim 1 as active ingredient.

4. Use of the compound of claim 1 in the preparation of an anti-prostate hypertrophy medicament.

5. Use of the pharmaceutical composition of claim 3 in the preparation of an anti-prostate hypertrophy medicament.

6. The pharmaceutical composition of claim 3, **characterized in that** the dosage form is selected form the group consisting of tablets, granules, capsules and oral solutions.
